# EUROPÄISCHE PATENTSCHRIFT

(11) **EP 2 654 620 B1**
(45) Veröffentlichungstag und Bekanntmachung des Hinweises auf die Patenterteilung: **14.02.2018**
(21) Anmeldenummer: 11822821.2
(22) Anmeldetag: 20.12.2011
(51) Int. Cl.: A61F 2/07

(54) **STENT-GRAFT**
STENT GRAFT
ENDOPROTHÈSE COUVERTE

(30) Priorität: 22.12.2010 DE 102010055545; 14.10.2011 DE 102011115902
(43) Veröffentlichungstag der Anmeldung: 30.10.2013
(73) Patentinhaber: Bentley InnoMed GmbH, 72379 Hechingen (DE)
(72) Erfinder: OBRADOVIC , Milisav, 79539 Lörrach (DE); BREGULLA, Rainer, 72336 Balingen (DE)
(74) Vertreter: Thiel, Christian
(86) Internationale Anmeldenummer: PCT/EP2011/006440
(87) Internationale Veröffentlichungsnummer: WO 2012/084202

(56) Entgegenhaltungen:
- EP-A2- 1 743 603
- WO-A2-01/66035

## Beschreibung

Die Erfindung betrifft einen Stent-Graft aus einem Stent mit einer Mehrzahl von nebeneinander angeordneten und miteinander verbundenen Ringsegmenten und wenigstens einer Membran. Ferner betrifft die Erfindung die Verwendung eines solchen Stent-Grafts zur Behandlung vaskulärer Fehlbildungen.

Stent-Grafts dieser Art werden in Blutgefäßen eingesetzt, etwa um anomal verengte, erweiterte oder auch geschädigte Blutgefäße zu stützen. Die Kombination von Stent und Membran dient dabei der Versorgung auch ausgedehnterer, behandlungsbedürftiger Gefäßabschnitte, welche eine größere Länge und vor allem auch Flexibilität des Implantats voraussetzen. Insbesondere werden Stent-Grafts bei der Überbrückung von Gefäßfehlbildungen eingesetzt, etwa um Aneurysmen von der Blutzirkulation auszuschließen. Zur Implantation solcher Stent-Grafts werden in der Regel Ballonkatheter verwandt.

Im Stand der Technik ist es bekannt, für diese Aufgabe Stent-Grafts einzusetzen, welche aus zwei Stents und einer flexiblen Membran, beispielsweise aus Teflon bestehen. Einen solchen Stent-Graft beschreibt die Druckschrift EP 2 151 217 A1. Der offenbarte Stent-Graft besteht aus einem inneren Stent und einem koaxial um diesen ersten angeordneten äußeren Stent, zwischen welchen eine flexible, dehnbare Membran angeordnet ist. Die Endbereiche der Stents mit der dazwischen angeordneten Membran werden verschweißt.

Die WO 01/66 035 A2 beschreibt einen Stent-Graft aus einem Stent mit einer Mehrzahl von nebeneinander angeordneten und miteinander verbundenen Ringsegmenten und wenigstens einer Membran, bei dem in der Membran zwischen einem Ringsegment und von diesem Ringsegment ausgehenden Verbindungstegen zum benachbarten Ringsegment angeordnet ist.

Die EP 1 266 635 A2 offenbart einen Stent-Graft, welcher einen zylindrischen Stent und eine zylindrische Membran aufweist, die beispielsweise über Nähte oder Haken miteinander verbunden sind. Zusätzlich oder alternativ kann die Verbindung durch ein geringes Überlappen von Stent und Membran gesichert werden.

Die WO 2009/035679 A1 offenbart einen Stent-Graft, welcher einen durchgehenden inneren Liner aus Polyester oder ePTFE aufweist. An einem Bereich dieses inneren Liners ist ein Stent angeordnet, welcher den inneren Liner koaxial umschließt. Die Bereiche des inneren Liners neben dem Stent sind mit einer zweiten Schicht aus Polyester oder ePTFE ummantelt, um die Wandstärke des Implantats in dem nicht durch den Stent gestützten Bereich zu erhöhen. Die Endbereiche von Stent und zweiter Schicht werden aneinander geschoben. Wenn erforderlich kann zusätzlich Verstärkungsmaterial, z. B. ePTFE, auf die Außenseite der Stent-Graft-Komponenten, insbesondere an den Übergängen zwischen Stent und zweiter Schicht, aufgebracht werden.

Von den genannten Stent-Grafts mit einer Verbindung von Membran und einem Stent verbindet lediglich der in der EP 1 266 635 A2 gezeigte genau einen Stent mit genau einer Membran. Die anderen Lösungen im Stand der Technik erfordern stets einen weiteren Stent oder eine weitere Membran, welche zur Verstärkung koaxial um den ersten Stent bzw. die erste Membran angeordnet sind.

Die Lösung gemäß der EP 1 266 635 A2 verwendet für die Verbindung zwischen Stent und Membran Haken oder Fäden und/oder alternativ ein Überlappen beider Bestandteile. Problematisch ist dabei stets die Haltbarkeit der Haken und Fäden, welche großen Reibungskräften innerhalb des Blutgefäßes ausgesetzt sind. Ebenso besteht die Gefahr der Gewebereizung oder -verletzung durch abstehende Haken oder überstehende Kanten zwischen Stent und Membran.

Es ist daher Aufgabe der vorliegenden Erfindung, einen Stent-Graft zu schaffen, welcher Stent und Membran einfach und haltbar miteinander verbindet, wenig Gefäßlumen verbraucht und keine Gewebereizung oder -verletzung verursacht.

Diese Aufgabe löst die Erfindung mit dem Stent-Graft der eingangs bezeichneten Art, mit wenigstens einem randständigen Ringsegment mit mäandrierendem Stegverlauf, bei dem einwärts- oder auswärts weisende Stegschlingen eingeschnitten sind, dergestalt, dass Federzungen entstehen, die formschlüssig in den Stegschlingen angeordnet sind und gegen den Stegverlauf federnd beweglich sind, wobei die Membran zwischen Federzungen und Steg eingeklemmt ist.

Der erfindungsgemäße Stent-Graft besteht aus einer Mehrzahl von miteinander verbundenen Ringsegmenten, die im mittleren Bereich angeordnet sind. Diese Ringsegmente entsprechen den Ringsegmenten herkömmlicher Stents, wie sie vielfach vorgeschlagen und in Gebrauch sind. Erfindungsgemäß ausgestaltet und verändert ist wenigstens ein randständiges Ringsegment, das einen mäandrierenden Stegverlauf aufweist und in dessen Stegschlingen Federzungen eingeschnitten sind.

Unter randständigen Ringsegmenten werden solche Ringsegmente verstanden, die am Rand der Membran angeordnet sind. Zumeist werden sie entweder endständig am Stent angeordnet sein, also den Stent an seinen Enden begrenzen, oder in unmittelbarer Nachbarschaft zu den endständigen Ringsegmenten angeordnet sind. Vorzugsweise handelt es sich um endständige Ringsegmente. Dabei kann erfindungsgemäß nur eines dieser randständigen Ringsegmente mit Federzungen versehen sein, vorzugsweise sind es allerdings randständige Ringsegmente an beiden Stentenden.

Unter mäandrierendem Stegverlauf der randständigen Ringsegmente wird sowohl ein wellenförmiger Stegverlauf als auch ein zickzackförmiger Stegverlauf verstanden. Wellenförmige und zickzackförmige Stegverläufe und Ringsegmente sind bei vaskulären Stents weitgehend üblich, um die bei der Expansion auftretende Längenkontraktion zumindest teilweise auszugleichen.

Zur Ausbildung der Federzungen werden die Stegschlingen der randständigen Ringsegmente partiell eingeschnitten, so dass sich gegen den Stegverlauf bewegliche Federzungen ausbilden. Dabei können entweder die einwärts- oder auswärtsweisenden Stegschlingen eingeschnitten sein, so dass sich einwärts- oder auswärtsweisende Federzungen ausbilden. Die Federzungen selbst sind im Fertigungszustand des Stents (also vor dem Aufkrimpen auf einen Ballon bzw. vor der Expansion) formschlüssig in die Stegschlingen eingepasst.

Um durch die Einschnitte in die Stegschlingen die Stegfestigkeit nicht zu verschlechtern, ist es angebracht, die Stegbreite im Bereich der Ausbildung der Federzungen zu erhöhen, etwa zu verdoppeln. Dies bedeutet, dass die Federzungen eine im Wesentlichen normale Stegbreite aufweisen.

Die Befestigung der Membran mit Hilfe von nebeneinander an dem Stent angeordneten Federzungen ermöglicht eine einfache und sichere Verbindung von Stent und Membran. Die in den Stegschlingen angeordneten Federzungen werden beispielsweise durch Laserschneiden erzeugt. Das Einklemmen der Membran unter die Federzungen ist fertigungstechnisch leicht zu bewerkstelligen. Die Integration der Verbindungselemente zwischen Stent und Membran als Federzungen in den Stent beseitigt dabei die Gefahr, das sich Verbindungsbestandteile vom Stent lösen und frei beweglich in die Blutbahn gelangen, wie dies bei anderen Verbindungselementen vorkommen kann.

Zur Erzeugung einer gleichmäßigen Verbindung zwischen Stent und Membran ist es sinnvoll, eine größere Anzahl von nebeneinander in die gleiche Richtung weisenden Federzungen anzuordnen. Insbesondere sind alle in die gleiche Richtung weisenden Stegschlingen des jeweiligen randständigen Ringsegments mit einer Federzunge ausgestattet. Diese Federzungen sind dann parallel zur Längsachse des Stents ausgerichtet und weisen vorzugsweise stenteinwärts. Die Form der Federzungen entspricht dabei weitgehend der Form der Stegschlingen.

In der Regel sind die Federzungen parallel zur Längsachse des Stents ausgerichtet. Bei entsprechender Gestaltung der Einschnitte und Anlage der Randsegmente bzw. Stegschlingen sind aber auch Abweichungen von der Parallelität möglich.

Die Anzahl der Federzungen eines randständigen Ringsegments richtet sich grundsätzlich nach der gewünschten Festigkeit der jeweiligen Verbindung zwischen Stent und Membran. In der Regel werden mindestens zwei gegenüberliegende Stegschlingen eines randständigen Ringsegments mit Federzungen ausgestattet sein, vorzugsweise alle Stegschlingen des randständigen Ringsegments.

Der erfindungsgemäße Stent-Graft wird in der Regel einen mittels eines Ballonkatheters aufweitbaren Stent, hergestellt beispielsweise aus einem medizinisch akzeptablen Stahl, aufweisen. Alternativ sind auch solche Varianten denkbar, bei welchen der Stent selbstexpandierend ausgebildet ist, z. B. durch Verwendung einer Formgedächtnislegierung wie Nitinol.

Die Membran, in der Regel eine Folie oder ein Schlauch, kann aus allen in der Medizintechnik üblichen und zugelassenen Materialien bestehen. Insbesondere eignen sich aber PTFE und Polyester. Besonders bevorzugt ist eine Membran aus ePTFE. Die Membran kann dabei zusätzlich funktionell beschichtet sein, z. B. mit entzündungshemmenden, proliferationshemmenden oder therapeutischen Stoffen, beispielsweise mit Rapamycin, Paclitaxel oder Heparin. Die Membran ist vorzugsweise schlauchförmig ausgebildet und weist das notwendige Dehnungsvermögen auf, um bei der Stentexpansion mitzugehen.

Das oder die randständigen Ringsegmente mit mäandrierendem Stegverlauf und die integrierten Federzungen sind insbesondere gleichzeitig die endständigen Ringsegmente des Stentteils des erfindungsgemäßen Stent-Grafts.

Die Federzungen der randständigen Ringsegmente mit mäandrierendem Stegverlauf weisen vorzugsweise stenteinwärts. Die zwischen den Stegschlingen und Federzungen eingeklemmte Membran kann zur Verbesserung des Sitzes im Endbereich, der unter die Federzungen zu liegen kommt, auf sich selbst zurückgefaltet sein, um einen besseren Klemmeffekt zu erzielen.

Der erfindungsgemäße Stent-Graft kann die Membran innen oder außen aufweisen. Bevorzugt ist eine Anordnung der Membran an der Außenseite. Diese Anordnung hat den Vorteil, dass die Einwirkung der Stentstruktur auf die Gefäßwand durch die zwischenliegende Membran gemildert wird. Insbesondere ist die Membran an beiden Enden des Stents in den randständigen Ringsegmenten festgeklemmt.

Möglich ist ferner eine Variante, bei der die Membran innenliegend im Stent in den Federzungen eingeklemmt ist, außen über die Stentoberfläche läuft und am entgegengesetzten Ende wiederum innenliegend von den Federzungen gehalten wird. In diesem Fall weisen die Federzungen vorzugsweise nach außen.

Vorzugsweise ist die Membran zusätzlich durch Verkleben an randständigen Ringsegmenten festgelegt. Dies kann durch einen körperverträglichen Klebstoff erfolgen, vorzugsweise aber über ein Klebefand, das in der Höhe der Federzungen über die Membran geklebt wird. Ein solches Klebeband dient zusätzlich zu dem Sicherungsaspekt auch dazu, die Gefäßwand vor dem direkten Kontakt mit den Federzungen des randständigen Ringsegments zu schützen.

Die Stent-Grafts gemäß der Erfindung können die Membran an beliebiger Stelle und auch mehr als eine Membran aufweisen. Beispielsweise kann die Membran nur an einem oder am anderen Ende des Stents oder mittig im Stent angeordnet sein und Stent-Bereiche frei lassen. Des Weiteren ist es möglich, mehr als nur eine Membran vorzusehen, die beispielsweise mit einem frei bleibenden Zwischenraum an den Enden angeordnet sind. Wenn mehr als nur eine Membran vorhanden ist, ist jede für sich über entsprechende Federzungen und ggf. durch Verkleben am Stentgerüst festgelegt.

Sind beispielsweise zwei Membranen endständig angeordnet, kann der dazwischenliegende nicht von einer Membran abgedeckte Teil des Stent-Grafts im Bereich einer Gefäßabzweigung angeordnet werden, so dass der Blutfluss in das abzweigende Gefäß nicht beeinträchtigt wird. Zu diesem Zweck können auch die beiden Stenthälften, die die Membran aufweisen, durch sogenannten Verbinder nur lose miteinander verbunden sein. Dies bringt zudem eine erhöhte Flexibilität in diesen Bereich mit sich.

Die erfindungsgemäßen Stent-Grafts werden in erster Linie zur Behandlung vaskulärer Fehlbildungen eingesetzt. Dabei kann es sich um das Abschließen von abzweigenden Gefäßen handeln, aber auch um das Stilllegen von Aneurysmen oder artheriovenösen Shunts.

Möglich sind ferner Ausführungsformen, bei denen der erfindungsgemäße Stent-Graft zwei Stenteinheiten aufweist, die dazu dienen, eine dazwischenliegende Schlauchmembran in ein Gefäß einzuspannen. In diesem Fall weist der Stent die Klemmverbindung zur Festlegung der Membran nur an einem Ende auf; das andere Ende der Schlauchmembran ist mit dem zweiten Stent verbunden. Ein solcher Stent-Graft kann in großflächig geschädigte Gefäße implantiert werden, beispielsweise nach Obliteration der Epithelzellschicht eines Blutgefäßes.

Die Erfindung wird im Folgenden anhand der Abbildungen näher erläutert. Es zeigen:
- Fig. 1: einen erfindungsgemäß modifizierten Stent,
- Fig. 2: einen erfindungsgemäßen Stent-Graft,
- Fig. 3: einen weiteren erfindungsgemäßen Stent für die Bespannung mit zwei Membranen und
- Fig. 4: einen erfindungsgemäßen Stent zur Bespannung mit zwei Membranen und einer flexiblen Verbindung der beiden Stentteile.

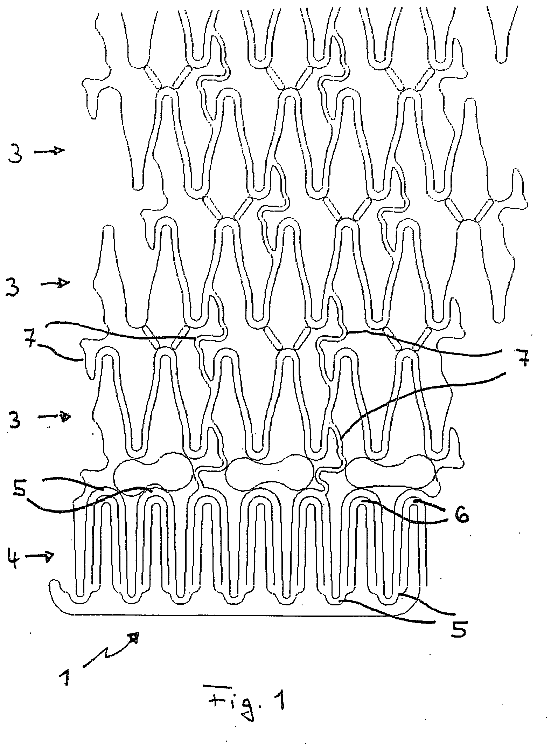

Fig. 1 zeigt einen erfindungsgemäß modifizierten Stent 1, wie er für die Festlegung einer Membran über Federzungen 6 modifiziert ist. Der Stent 1 besteht aus einer Mehrzahl von Ringsegmenten 3, die ein im Wesentlichen zickzackförmigen Verlauf haben. In der Abbildung ist der Stent 1 in aufgeschnittenem und ausgebreitetem Zustand gezeigt; im Originalzustand nach der Fertigung ist er ein aus Stegen zusammengesetztes und mit Durchbrechungen versehenes Rohr, beispielsweise aus einem medizinischem Stahl oder Nitinol. Der Fertigung erfolgt auf an und für sich bekannte Weise durch Laserschneiden eines geeignet dimensionierten Rohres.

Die Ringsegmente 3 sind durch Dehnungselemente 7 miteinander verbunden dergestalt, dass bei Platzierung des Stents über einen Ballon die Längenreduktion, die sich aus der Expansion der Ringsegmente 3 ergibt, zumindest teilweise durch eine Streckung der Verbindungselemente 7 kompensiert wird.

Das randständige Ringsegment 4 am Stentende hat einen mäandrierenden Verlauf. Ein zickzackförmiger Verlauf wäre ebenfalls möglich. Die einzelnen Stege bilden Stegschlingen 5 aus, die wellen- oder mäanderförmig über die Zirkumferenz des Stents verlaufen. Die einzelnen Stegschlingen 5 weisen dabei im Verlauf ihrer stenteinwärts weisenden Zungen Einschnitte 8 auf, die Federzungen 6 entstehen lassen, welche federnd gegen den generellen Verlauf der Stege der randständigen Ringsegmente 4 beweglich sind. Dies ermöglicht es, zwischen die Federzungen 6 und die Stegschlingen 5 eine Membran einzuschieben, die dort klemmend festgehalten wird.

Es ist festzuhalten, dass für einen erfindungsgemäßen Stent-Graft die Struktur des Stents selbst nicht entscheidend ist. Die Ringsegmente können unterschiedlich aufgebaut sein. Wichtig ist, dass ein randständiges Ringsegment vorhanden ist, in das Federzungen eingeschnitten sind, um eine Membran darin festzulegen.

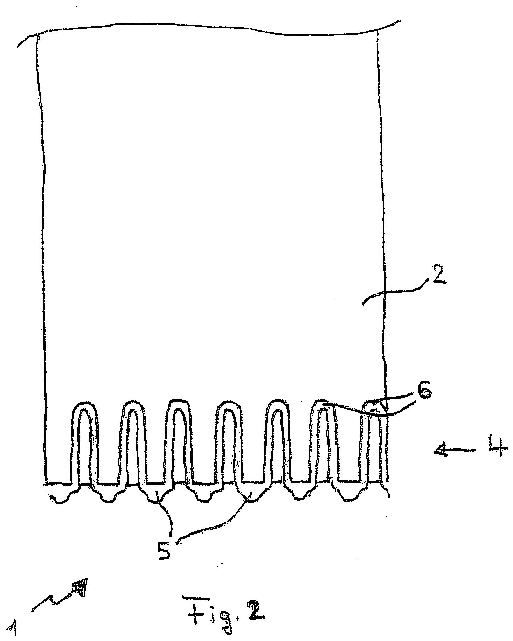

Fig. 2 zeigt einen erfindungsgemäßen Stent-Graft mit einem Stent gemäß Fig. 1 und eingelegter Membran 2. Zu sehen sind die endseitigen Stentschlingen 5 wie auch die Federzungen 6, die auf der Membran 2 zu liegen kommen. Im Falle einer Sicherung der Membran auf dem Stent mit einem Klebemittel wird zu diesem Zwecke ein Klebeband über die Federzungen 6 bei abgelegter Membran geklebt.

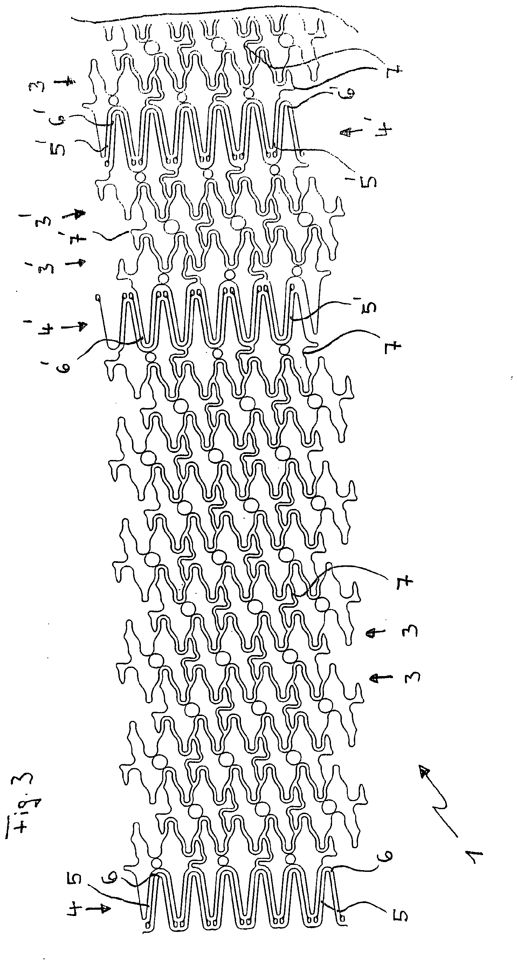

Fig. 3 zeigt einen erfindungsgemäß modifizierten Stent 1, der sich in zwei Teile gliedert, die jeweils für sich durch Membranen abdecken lassen. Im linken Teil sind die randständigen Ringsegmente 4 mit Stegschlingen 5 und Federzungen 6 ausgestattet. Daran anschließend folgen eine Reihe von Ringsegmenten, die jeweils über Verbindungs- oder Dehnungselemente 7 miteinander verbunden sind. Im zentralen Bereich der Stents folgt ein weiteres "randständiges" Ringsegment 4' mit Stegschlingen 5' und Federzungen 6'. Es folgt ein mittig im Stent 1 angelegter Übergangsbereich mit Ringsegmenten 3' und Dehnungselementen 7', an denen sich ein weiteres "randständiges" Ringsegment 4' mit Stegschlingen 5' und Federzungen 6' anschließt. Es folgen Ringsegmente 3, die über Dehnungselemente 7 verbunden sind und (nicht mehr dargestellt) das andere Ende des Stents 1 mit einem weiteren randständigen Ringsegment 4 mit Stegschlingen 5 und Federzungen 6.

Die Federzungen 6 und 6' der linksseitig dargestellten Stenthälfte weisen im dargestellten Fall zueinander und nehmen die schlauchförmige Membran zwischen sich auf. Entsprechendes gilt für den rechtseitigen Teil des Stents 1.

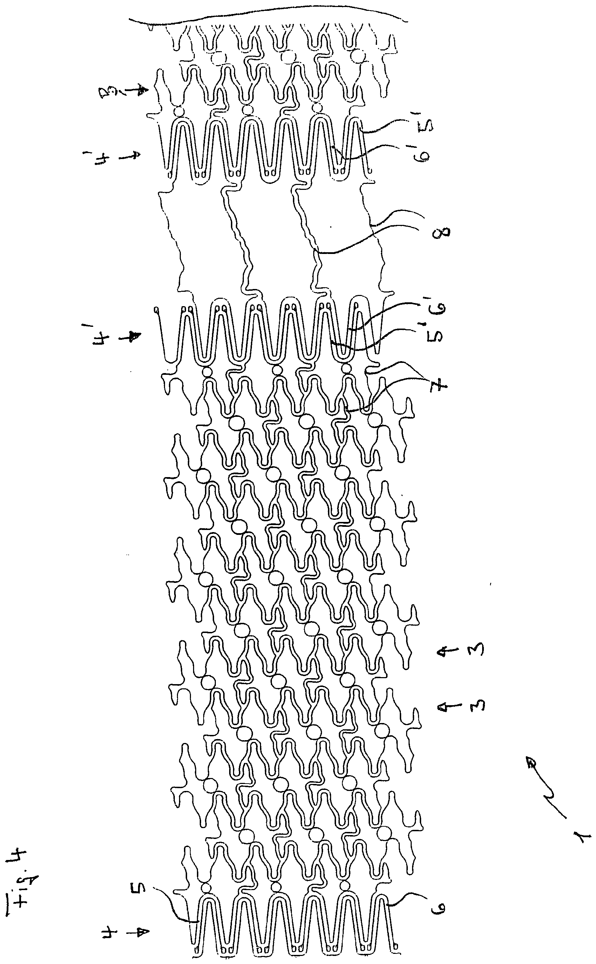

Fig. 4 zeigt eine weitere Ausführungsform eines erfindungsgemäßen Stents für die Herstellung eines Stent-Grafts mit zwei Membranen. In diesem Fall ist der Stent 1 ebenfalls an beiden Enden mit randständigen Ringsegmenten 4 ausgestattet (linksseitiges Ende gezeigt), die wiederum Stegschlingen 5 und Federzungen 6 aufweisen. Die Federzungen 6 sind stenteinwärts gerichtet. Im Übrigen weist der Stent die üblichen Ringsegmente 3 auf, die den im fertigen Stent-Graft von der Membran abgedeckten linken Teil des Stents zwischen den randständigen Segmenten 4 und 4' abdeckt. Die einzelnen Ringsegmente 3 und 4 sind über Verbindungselemente 7 miteinander verbunden.

Wie die randständigen Ringsegmente 4 weisen auch die zentral angeordneten randständigen Ringsegmente 4' Federzungen 6' und Stegschlingen 5' auf.

Die rechte Stenthälfte, nur mit dem linksseitigen Ende dargestellt, entspricht in allen Punkten der linken Stenthälfte. Die beiden Stenthälften sind durch Verbinder 8 aneinander gekoppelt. Im dargestellten Fall weist der Stent 1 insgesamt drei Verbinder 8 auf. Die Verbinder lassen zwischen den beiden Stenthälften einen größeren Freiraum, was es zum einen erlaubt, abgehende Gefäße freizuhalten, zum anderen aber auch eine erhöhte Flexibilität mit sich bringt.

Abgesehen von den Verbindern 8 bzw. den Ringsegmenten 3' und Verbindungselementen 7' entspricht der Stent von Fig. 4 in allen weiteren Punkten dem von Fig. 3.

Er erfindungsgemäße Stent-Graft wird in üblicher Weise auf einen Ballonkatheter gecrimpt und mit üblicher Technik am Einsatzort expandiert. Dabei legt sich der Stent 1 mit der Membran 2 an die Gefäßwand an. Die Membran 2 befindet sich zwischen Stent 1 und Gefäßwand.

## Patentansprüche

1. Stent-Graft aus einem Stent (1) mit einer Mehrzahl von nebeneinander angeordneten und miteinander verbundenen Ringsegmenten (3) und wenigstens einem randständigen Ringsegment (4) mit mäandrierendem Stegverlauf sowie wenigstens einer Membran (2), **dadurch gekennzeichnet, dass** bei wenigstens einem randständigen Ringsegment (4) mit mäandrierendem Stegverlauf einwärts- oder auswärtsweisende Stegschlingen (5) eingeschnitten sind, in der Gestalt, dass Federzungen (6) entstehen, die formschlüssig in den Stegschlingen (5) angeordnet sind und gegen den Stegverlauf federnd beweglich sind, wobei die Membran (2) zwischen Federzungen (6) und Stegschlingen (5) eingeklemmt ist.

2. Stent-Graft nach Anspruch 1, **dadurch gekennzeichnet, dass** wenigstens ein randständiges Ringsegment (4) endständig angeordnet ist.

3. Stent-Graft nach Anspruch 1 oder 2, **dadurch gekennzeichnet, dass** die Federzungen (6) stenteinwärts weisen.

4. Stent-Graft nach einem der vorhergehenden Ansprüche, **dadurch gekennzeichnet, dass** der Stent (1) eine Membran (2) in Form eines Schlauches aufweist.

5. Stent-Graft nach Anspruch 4, **dadurch gekennzeichnet, dass** die Membran (2) den Stent (1) umgibt.

6. Stent-Graft nach einem der vorstehenden Ansprüche, **dadurch gekennzeichnet, dass** die Membran (2) durch Verkleben vorzugsweise mit Klebeband in den Federzungen (6) gesichert ist.

7. Stent-Graft nach einem der Ansprüche 1 bis 6, **gekennzeichnet, durch** mehrere abschnittsweise angeordnete Membranen (2), die jeweils an Federzungen (6) gesichert sind.

8. Stent-Graft nach einem der vorstehenden Ansprüche, **dadurch gekennzeichnet, dass** der Randbereich der in den Federzungen (6) eingeklemmten Membran (2) umgeschlagen ist.

9. Stent-Graft nach einem der vorstehenden Ansprüche, **dadurch gekennzeichnet, dass** die Membran (2) aus ePTFE-Schlauch besteht.

10. Stent-Graft nach einem der vorstehenden Ansprüche, **dadurch gekennzeichnet, dass** der Stent (1) ein Ballon-expandierbarer Stent ist.

11. Stent-Graft nach Anspruch 10, aufgekrimpt auf den Ballon eines Ballonkatheters.

12. Stent-Graft nach einem der Ansprüche 1 bis 9 mit einem selbstexpandierenden Stent aus einer Formgedächtnislegierung, insbesondere Nitinol.

13. Verwendung eines Stent-Grafts nach einem der Ansprüche 1 bis 12 zur Behandlung vaskulärer Fehlbildungen.

## Claims

1. Stent graft composed of a stent (1) having a plurality of ring segments (3) disposed next to one another and connected with one another, at least one edge-positioned ring segment (4) having a meander-shaped strut progression, and at least one membrane (2), **characterized in that** at least one edge-positioned ring segment (4) having a meander-shaped strut progression comprises strut loops (5) that face inward or outward and that are cut in such a manner that spring tongues (6) are formed, which are disposed in the strut loops (5) with shape fit, and can be resiliently moved counter to the strut progression, wherein the membrane is clamped in between spring tongues (6) and strut.

2. Stent graft according to claim 1, **characterized in that** at least one edge-positioned ring segment (4) is disposed to be end-positioned.

3. Stent graft according to claim 1 or 2, **characterized in that** the spring tongues (6) point stent-inward.

4. Stent graft according to one of the preceding claims, **characterized in that** the stent (1) has a membrane (2) in the form of a tube.

5. Stent graft according to claim 4, **characterized in that** the membrane (2) surrounds the stent (1).

6. Stent graft according to one of the preceding claims, **characterized in that** the membrane (2) is secured in the spring tongues (6) by means of gluing, preferably using adhesive tape.

7. Stent graft according to one of claims 1 to 6, **characterized by** multiple membranes (2) disposed in certain sections, which are secured on spring tongues (6), in each instance.

8. Stent graft according to one of the preceding claims, **characterized in that** the edge region of the membrane (2) clamped into the spring tongues (6) is folded over.

9. Stent graft according to one of the preceding claims, **characterized in that** the membrane (2) consists of an ePTFE tube.

10. Stent graft according to one of the preceding claims, **characterized in that** the stent (1) is a balloon-expandable stent.

11. Stent graft according to claim 10, crimped onto the balloon of a balloon catheter.

12. Stent graft according to one of claims 1 to 9, having a self-expanding stent composed of a shape memory alloy, particularly nitinol.

13. Use of a stent graft according to one of claims 1 to 12 for the treatment of vascular malformations.

## Revendications

1. Greffe d'endoprothèse constituée d'une endoprothèse (1) ayant une pluralité de segments annulaires (3) disposés les uns à côté des autres et connectés les uns aux autres et au moins un segment annulaire marginal (4) avec une allure de nervure en méandres ainsi qu'au moins une membrane (2), **caractérisée en ce que** dans le cas d'au moins un segment annulaire marginal (4) avec une allure de nervure en méandres, des boucles de nervure tournées vers l'intérieur ou vers l'extérieur (5) sont entaillées de de manière à obtenir des langues à ressort (6) qui sont disposées dans les boucles de nervure (5) par engagement par correspondance de formes et qui peuvent être déplacées élastiquement à l'encontre de l'allure de la nervure, la membrane (2) étant serrée entre les langues à ressort (6) et les boucles de nervure (5).

2. Greffe d'endoprothèse selon la revendication 1, **caractérisée en ce qu'**au moins un segment annulaire marginal (4) est disposé à l'extrémité.

3. Greffe d'endoprothèse selon la revendication 1 ou 2, **caractérisée en ce que** les langues à ressort (6) sont tournées vers l'intérieur de l'endoprothèse.

4. Greffe d'endoprothèse selon l'une quelconque des revendications précédentes, **caractérisée en ce que** l'endoprothèse (1) présente une membrane (2) en forme de tuyau.

5. Greffe d'endoprothèse selon la revendication 4, **caractérisée en ce que** la membrane (2) entoure l'endoprothèse (1).

6. Greffe d'endoprothèse selon l'une quelconque des revendications précédentes, **caractérisée en ce que** la membrane (2) est fixée dans les langues à ressort (6) par collage, de préférence avec une bande adhésive.

7. Greffe d'endoprothèse selon l'une quelconque des revendications 1 à 6, **caractérisée par** plusieurs membranes (2) disposées par sections, qui sont à chaque fois fixées aux langues à ressort (6).

8. Greffe d'endoprothèse selon l'une quelconque des revendications précédentes, **caractérisée en ce que** la région de bord de la membrane (2) serrée dans les langues à ressort (6) est repliée.

9. Greffe d'endoprothèse selon l'une quelconque des revendications précédentes, **caractérisée en ce que** la membrane (2) se compose d'un tuyau en ePTFE.

10. Greffe d'endoprothèse selon l'une quelconque des revendications précédentes, **caractérisée en ce que** l'endoprothèse (1) est une endoprothèse pouvant être déployée par un ballonnet.

11. Greffe d'endoprothèse selon la revendication 10, sertie sur le ballonnet d'un cathéter à ballonnet.

12. Greffe d'endoprothèse selon l'une quelconque des revendications 1 à 9, comprenant une endoprothèse auto-déployable constituée d'un alliage à mémoire de forme, en particulier de Nitinol.

13. Utilisation d'une greffe d'endoprothèse selon l'une quelconque des revendications 1 à 12 pour le traitement de malformations vasculaires.
